# FASCICULE DE BREVET EUROPEEN

(11) **EP 3 648 897 B1**
(45) Date de publication et mention de la délivrance du brevet: **01.03.2023**
(21) Numéro de dépôt: 18749020.6
(22) Date de dépôt: 03.07.2018
(51) Int. Cl.: B05B 1/28, B05B 11/02, A61M 11/00, A61M 15/00

(54) **DISPOSITIF DE DISTRIBUTION DE PRODUIT FLUIDE.**
VORRICHTUNG ZUR AUSGABE EINES FLÜSSIGPRODUKTS
DEVICE FOR DISPENSING FLUID PRODUCT

(30) Priorité: 06.07.2017 FR 1756365
(43) Date de publication de la demande: 13.05.2020
(73) Titulaire: Aptar France SAS, 27110 Le Neubourg (FR)
(72) Inventeur: BROUET, Guillaume, 76000 Rouen (FR)
(74) Mandataire: CAPRI
(86) Numéro de dépôt international: PCT/FR2018/051641
(87) Numéro de publication internationale: WO 2019/008260

(56) Documents cités:
- DE-A1- 1 425 861
- US-A- 5 348 189
- US-A1- 2008 210 229
- US-A1- 2017 129 662
- US-B2- 9 592 934

## Description

La présente invention concerne un dispositif de distribution de produit fluide.

Plus particulièrement, la présente invention concerne un dispositif de distribution de produit fluide pour distribuer un produit fluide pharmaceutique à un utilisateur, par exemple par l'intermédiaire d'une pulvérisation nasale. La présente invention s'applique toutefois également aux dispositifs de distribution de parfum ou de produits cosmétiques. Dans ce type de dispositif, le produit fluide, généralement liquide, est distribué ou pulvérisé à travers un orifice de distribution lors de l'actionnement manuel de ce dispositif. Après chaque actionnement, il peut rester du produit fluide au niveau de l'orifice de distribution ainsi qu'à l'intérieur des canaux et passages qui relient le réservoir audit orifice de distribution. En particulier dans le domaine pharmaceutique, ceci peut poser des problèmes. D'une part, ces résidus peuvent sécher et obturer l'orifice et/ou les passages de fluide et donc empêcher un actionnement ultérieur du dispositif. D'autre part, ces volumes résiduels de produit actifs peuvent être source de contamination. En particulier dans des dispositifs de type bidose, c'est-à-dire adaptés à distribuer deux doses, si la deuxième dose n'est pas distribuée immédiatement après la première, il y a un risque de contamination de cette seconde dose. De plus, dans le cas de substances fortement actives, telles que par exemple le Fentanyl, qui est un produit utilisé pour le traitement de la douleur cent fois plus puissant que la morphine, il est nécessaire d'éviter les risques d'utilisation du volume résiduel par un tiers pour lequel les conséquences seraient dramatiques. Lors d'utilisations dans le cadre hospitalier, cela est géré par des professionnels, mais il y a maintenant un nombre important de produits de ce type qui sont utilisés directement chez le patient, d'où une exposition au risque plus grande. Les autorités réglementaires sont très sensibles à cet aspect et demandent systématiquement aux laboratoires de mettre en place des moyens pour réduire l'accès au volume résiduel.

Les documents US9592934, DE1425861, US 2008/210229 A1, US 5 348 189 A et US2017129662 décrivent des dispositifs de l'état de la technique.

La présente invention a pour but de fournir un dispositif de distribution de produit fluide qui ne reproduit pas les inconvénients susmentionnés.

Plus particulièrement, la présente invention a pour but de fournir un dispositif de distribution de produit fluide qui évite ou limite les risques de bouchage du dispositif, ainsi que les risques de contamination du produit fluide.

La présente invention a aussi pour but de fournir un tel dispositif qui empêche ou limite l'accès aux volumes résiduels de produit fluide après utilisation.

La présente invention a également pour but de fournir un tel dispositif qui soit simple et peu coûteux à fabriquer et à assembler.

La présente invention a donc pour objet un dispositif de distribution de produit fluide, comportant un réservoir contenant du produit fluide, une tête de distribution pourvue d'un orifice de distribution, des moyens de distribution pour distribuer au moins une partie dudit produit fluide à travers ledit orifice de distribution, et des moyens de passage pour relier ledit réservoir audit orifice de distribution lors de l'actionnement desdits moyens de distribution, ledit dispositif comportant au moins un insert poreux réalisé en matériau poreux, pour aspirer, notamment par capillarité, et piéger au moins partiellement le produit fluide résiduel après actionnement, ledit réservoir comportant un bouchon qui obture ledit réservoir de manière étanche avant actionnement.

Selon l'invention, lesdits moyens de passage comportent une aiguille adaptée à percer ledit bouchon lors de l'actionnement.

Avantageusement, ladite aiguille est fixe par rapport audit orifice de distribution.

Avantageusement, ledit insert poreux est réalisé en polymère poreux hydrophile.

Selon un premier mode de réalisation avantageux, ledit insert poreux est disposé au moins partiellement autour desdits moyens de passage.

Avantageusement, après actionnement, ledit insert poreux s'étend au moins partiellement dans ledit réservoir.

Avantageusement, un insert rigide proximal est disposé en aval dudit insert poreux.

Avantageusement, ledit insert rigide proximal définit un profil de pulvérisation directement en amont dudit orifice de distribution.

Avantageusement, un insert rigide distal est disposé en amont dudit insert poreux.

Avantageusement, ledit insert poreux est disposé axialement entre ledit insert rigide proximal et ledit insert rigide distal.

Selon un second mode de réalisation avantageux, ledit insert poreux est disposé dans ledit réservoir.

Avantageusement, ledit réservoir contient un second bouchon qui sépare ledit insert poreux du produit fluide contenu dans ledit réservoir.

Avantageusement, en fin d'actionnement, ladite aiguille perce ledit second bouchon pour relier ledit insert poreux auxdits moyens de passage.

Ces caractéristiques et avantages et d'autres de la présente invention apparaitront plus clairement au cours de la description détaillée suivante, faite en référence aux dessins joints, donnés à titre d'exemples non limitatifs, et sur lesquels:
La figure 1 est une vue schématique en section transversale d'un dispositif de distribution de produit fluide selon un premier mode de réalisation avantageux de la présente invention, en position de repos avant actionnement,
La figure 2 est une vue similaire à la figure 1, montrant le dispositif après actionnement,
La figure 3 est une vue de détail d'une variante de réalisation du dispositif des figures 1 et 2, en position de repos avant actionnement,
La figure 4 est une vue schématique en section transversale d'un dispositif de distribution de produit fluide selon un second mode de réalisation avantageux de la présente invention, en position de repos avant actionnement, et
La figure 5 est une vue similaire à la figure 4, montrant le dispositif après actionnement.

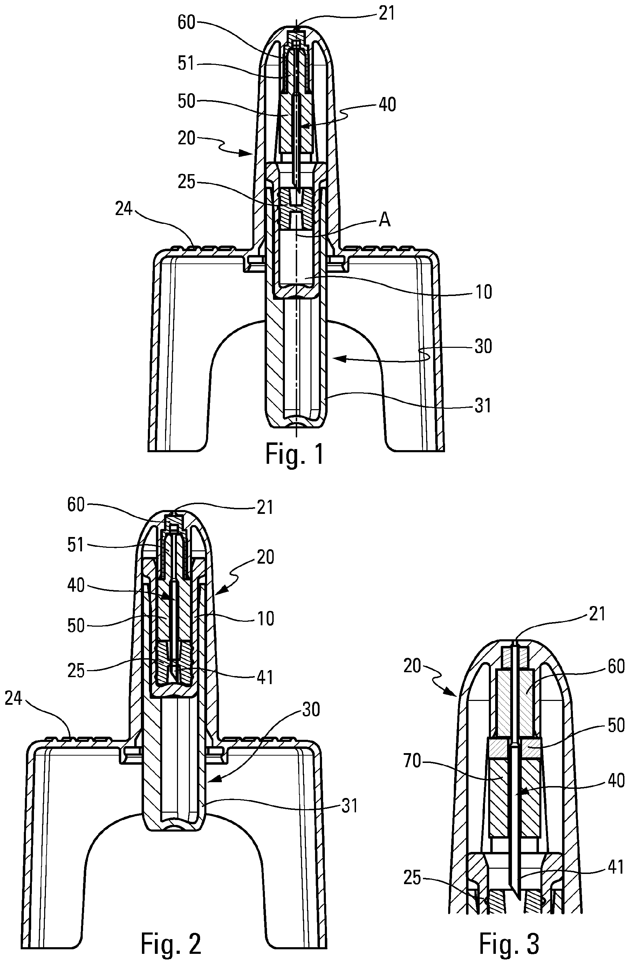

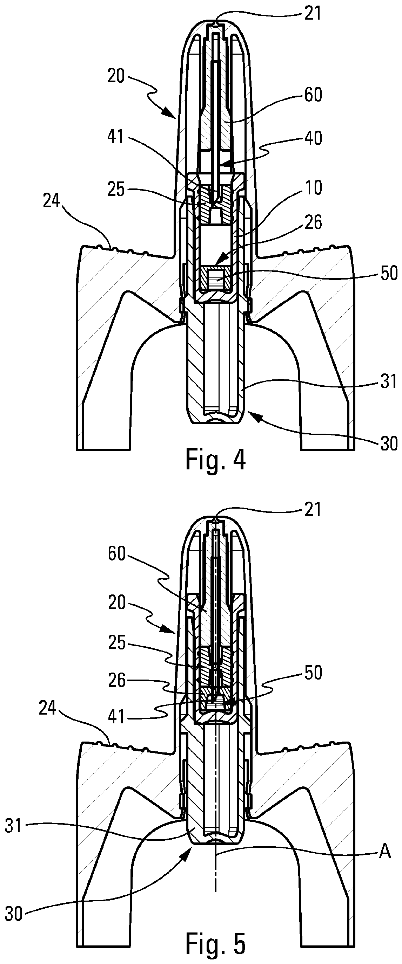

Les termes "proximal" et "distal" se réfèrent à l'orifice de distribution. Les termes "axial" et "radial" se réfèrent à l'axe central longitudinal A représenté sur les figures 1 et 5. Les termes "amont" et "aval" se réfèrent à la direction d'écoulement du fluide lors de l'actionnement.

En référence aux figures 1 et 2, il est représenté un premier mode de réalisation avantageux de la présente invention. Dans ce première mode de réalisation, un réservoir 10 contenant du produit fluide à distribuer, typiquement un liquide, est disposé à l'intérieur d'un corps formant une tête de distribution 20. Cette tête de distribution 20 comporte un orifice de distribution 21, qui dans l'exemple des figures, est orienté axialement, mais qui pourrait être orienté différemment, par exemple radialement. Cet orifice de distribution 21 sert à distribuer une dose de produit fluide hors de ladite tête de distribution 20 lors de l'actionnement du dispositif par un utilisateur, cette distribution étant réalisée par des moyens de distribution.

Le réservoir 10 est dans cet exemple formé par un corps creux et borgne, comportant une seule ouverture fermée par un bouchon 25 et contenant une seule dose de produit fluide à distribuer un actionnement unique du dispositif. Il est à noter que la présente invention pourrait aussi s'adapter à des dispositifs du type bidose, contenant deux doses de produit fluide à distribuer en deux actionnements successifs du dispositif.

La tête de distribution 20 comporte des moyens de passage 40 qui relient, en cours d'actionnement, le réservoir 10 à l'orifice de distribution 21. Ces moyens de passage 40 comportent avantageusement une aiguille 41 adaptée à transpercer le bouchon 25 au moment de l'actionnement. Cette aiguille 41 est creuse, et peut être formée de manière classique. De préférence, cette aiguille 41 est fixe par rapport à la tête de distribution 20 et notamment par rapport à l'orifice de distribution 21. En variante, cette aiguille pourrait être mobile par rapport à ladite tête de distribution 20.

D'autres moyens de passage sont envisageables, par exemple sans aiguille, tel que décrit par exemple dans le document WO9729028.

Des moyens d'actionnement 30 sont prévus pour permettre l'actionnement du dispositif. En l'occurrence, ces moyens d'actionnement 30 comportent un corps d'actionnement 31 mobile par rapport à la tête de distribution 20, ledit corps d'actionnement 31 coopérant avec ledit réservoir 10 pour le déplacer axialement par rapport à la tête de distribution 20 et donc par rapport à l'aiguille 41, en direction de l'orifice de distribution 21.

Selon l'invention, le dispositif comporte un insert 50 réalisé en matériau poreux pour aspirer par capillarité et piéger au moins partiellement le produit fluide résiduel après actionnement du dispositif, en particulier au niveau desdits moyens de passage 40 et/ou dudit orifice de distribution 21.

Dans le premier mode de réalisation des figures 1 et 2, ledit insert poreux 50 est disposé au moins partiellement autour desdits moyens de passage 40.

Cet insert 50 s'étend dans cet exemple autour de l'aiguille 41 et se prolonge en direction de l'orifice de distribution 21. Comme visible sur la figure 2, en fin d'actionnement, ledit insert 50 vient au contact du bouchon 25 dans le réservoir 10.

Un insert rigide proximal 60 est de préférence prévu en aval dudit insert poreux 50, pour définir un profil de pulvérisation directement en amont de l'orifice de distribution 21, afin de garantir une bonne pulvérisation du produit fluide lors de sa distribution. Dans l'exemple représenté sur les figures 1 et 2, ledit insert rigide proximal 60 est disposé partiellement autour d'une partie proximale de diamètre réduit 51 dudit insert poreux 50. En particulier, l'aiguille 41 peut s'étendre dans ledit insert poreux 50 jusqu'à ladite partie de diamètre réduit 51. L'insert poreux 50 est dans ce cas en contact du fluide à distribuer uniquement au niveau de ladite partie de diamètre réduit 51.

La figure 3 illustre une variante du premier mode de réalisation, dans laquelle en plus dudit insert rigide proximal 60, il est prévu un insert rigide distal 70, disposé en amont dudit insert poreux 50. Cette mise en oeuvre permet de mieux fixer l'aiguille 41 au niveau de l'insert rigide distal 70, réduisant la dimension dudit insert poreux 50, qui reste toutefois suffisante pour absorber le volume de fluide résiduel après actionnement. Avantageusement, ledit insert poreux 50 est disposé axialement entre lesdits inserts rigides proximal et distal 60, 70.

Les figures 4 et 5 illustrent un second mode de réalisation, dans lequel ledit insert poreux 50 est disposé au fond du réservoir 10. Un second bouchon 26 sépare ledit insert poreux 50 du produit fluide contenu dans le réservoir 10. A la fin de la distribution du produit fluide, comme visible sur la figure 5, l'aiguille 41 vient percer ce second bouchon 26 pour relier ledit insert poreux aux moyens de passage 40. Ceci va permettre audit insert poreux 50 d'aspirer, notamment par capillarité, au moins partiellement le produit fluide résiduel après actionnement.

Eventuellement, on pourrait combiner les deux modes de réalisation cidessus, et prévoir deux inserts poreux, l'un autour des moyens de passage 40, l'autre au fond du réservoir 10.

Avantageusement, ledit insert poreux 50 est réalisé en polymère poreux hydrophile, tel que le Porex^{®}. D'autres matériaux poreux équivalents sont aussi envisageables.

Le fonctionnement du dispositif représenté sur les figures va être détaillé ci-après.

De manière classique, dans le dispositif représenté sur les figures, l'utilisateur place deux doigts sur des appuis radiaux 24 formés sur la tête de distribution 20, et appuie avec le pouce sur le fond axial distal dudit corps d'actionnement 31. Lors d'un tel actionnement, le réservoir 10 est donc poussé axialement en direction de l'orifice de distribution 21, de sorte que l'aiguille 41 va transpercer le bouchon 25. Le contenu du réservoir 10 est alors relié à l'orifice de distribution 21 et l'appui de l'utilisateur sur le corps d'actionnement 31 déplace le bouchon 25 dans le réservoir 10 pour assurer la distribution du produit fluide. Ce bouchon 25 agit alors en tant que piston en refoulant le produit fluide hors dudit réservoir 10, à travers l'aiguille 41. Dans ce mode de réalisation, le bouchon 25 forme donc les moyens de distribution qui lors de l'actionnement assurent la distribution du produit fluide à travers l'orifice de distribution. En fin de distribution, le fluide résiduel susceptible de rester à l'intérieur du dispositif, dans lesdits moyens de passage 40 et/ou au niveau dudit orifice de distribution 21, sera au moins partiellement aspiré par ledit insert poreux 50, empêchant l'accès à ce volume de fluide résiduel piégé.

Dans un autre mode de réalisation, non représenté sur les dessins, le réservoir pourrait ne pas être formé par un corps creux borgne ne comportant qu'une ouverture, mais par un cylindre creux ouvert axialement des deux côtés. Ce cylindre serait alors fermé du côté proximal par un premier bouchon et du côté distal par un second bouchon, le volume défini entre lesdits deux bouchons contenant le produit fluide à distribuer. Lorsque l'utilisateur actionne le dispositif, il va comme décrit précédemment appuyer axialement sur le corps d'actionnement pour le faire coulisser axialement vers l'orifice de distribution. Ceci provoque le déplacement du second bouchon à l'intérieur du réservoir. Or, le produit fluide étant incompressible, ce déplacement du second bouchon va donc déplacer le premier bouchon en direction de l'aiguille, qui elle est fixe. Le premier bouchon va donc être percé par l'aiguille et le contenu du réservoir va être distribué à travers ladite aiguille par le second bouchon, qui agit alors en tant que piston. Dans ce mode de réalisation, c'est le second bouchon qui forme les moyens de distribution. Ce mode de réalisation pourrait incorporer un insert poreux tel que décrit précédemment en référence soit au premier mode de réalisation des figures 1 à 3, soit au second mode de réalisation des figures 4 et 5. Comme précédemment, en fin de distribution, le fluide résiduel susceptible de rester à l'intérieur du dispositif, dans lesdits moyens de passage et/ou au niveau dudit orifice de distribution, sera au moins partiellement aspiré par ledit insert poreux, empêchant l'accès à ce volume de fluide résiduel piégé.

Comme évoqué précédemment, l'invention pourrait aussi s'appliquer à un dispositif du type bidose. Dans ce cas, le contenu du réservoir serait distribué en deux actionnements successifs. Le document WO2014147329 décrit un exemple de dispositif bidose. Dans une telle variante, l'insert poreux selon le premier mode de réalisation des figures 1 à 3 empêcherait qu'entre les deux doses, du fluide résiduel soit contaminé et ensuite distribué lors du second actionnement. Bien entendu, comme précédemment, ledit insert poreux pourrait également empêcher l'accès au produit fluide résiduel piégé après chaque actionnement. Une combinaison des premier et second modes de réalisation serait ici avantageuse, en permettant avec un insert poreux disposé autour des moyens de passage de piéger le produit fluide résiduel après le premier actionnement et avec un autre insert poreux disposé dans le fond du réservoir de piéger le produit fluide résiduel après le second actionnement.

Bien entendu, d'autres variantes de réalisation sont également envisageables, sans sortir du cadre de la présente invention, tel que défini par les revendications annexées.

## Revendications

1. Dispositif de distribution de produit fluide, comportant un réservoir (10) contenant du produit fluide, une tête de distribution (20) pourvue d'un orifice de distribution (21), des moyens de distribution (25) pour distribuer au moins une partie dudit produit fluide à travers ledit orifice de distribution (21), et des moyens de passage (40) pour relier ledit réservoir (10) audit orifice de distribution (21) lors de l'actionnement desdits moyens de distribution (25), ledit dispositif comportant au moins un insert poreux (50) réalisé en matériau poreux, pour aspirer, notamment par capillarité, et piéger au moins partiellement le produit fluide résiduel après actionnement, **caractérisé en ce que** ledit réservoir (10) comporte un bouchon (25) qui obture ledit réservoir (10) de manière étanche avant actionnement, lesdits moyens de passage (40) comportant une aiguille (41) adaptée à percer ledit bouchon (25) lors de l'actionnement.

2. Dispositif selon la revendication 1, dans lequel ladite aiguille (41) est fixe par rapport audit orifice de distribution (21).

3. Dispositif selon l'une quelconque des revendications précédentes, dans lequel ledit insert poreux (50) est réalisé en polymère poreux hydrophile.

4. Dispositif selon l'une quelconque des revendications précédentes, dans lequel ledit insert poreux (50) est disposé au moins partiellement autour desdits moyens de passage (40).

5. Dispositif selon la revendication 4, dans lequel, après actionnement, ledit insert poreux (50) s'étend au moins partiellement dans ledit réservoir (10).

6. Dispositif selon la revendication 4 ou 5, dans lequel un insert rigide proximal (60) est disposé en aval dudit insert poreux (50).

7. Dispositif selon la revendication 6, dans lequel ledit insert rigide proximal (60) définit un profil de pulvérisation directement en amont dudit orifice de distribution (21).

8. Dispositif selon l'une quelconque des revendications 4 à 7, dans lequel un insert rigide distal (70) est disposé en amont dudit insert poreux (50).

9. Dispositif selon la revendication 8 et l'une des revendications 6 ou 7, dans lequel ledit insert poreux (50) est disposé axialement entre ledit insert rigide proximal (60) et ledit insert rigide distal (70).

10. Dispositif selon l'une quelconque des revendications 1 à 3, dans lequel ledit insert poreux (50) est disposé dans ledit réservoir (10).

11. Dispositif selon la revendication 10, dans lequel ledit réservoir (10) contient un second bouchon (26) qui sépare ledit insert poreux (50) du produit fluide contenu dans ledit réservoir (10).

12. Dispositif selon la revendication 11 et l'une des revendications 2 ou 3, dans lequel, en fin d'actionnement, ladite aiguille (41) perce ledit second bouchon (26) pour relier ledit insert poreux (50) auxdits moyens de passage (40).

## Patentansprüche

1. Vorrichtung zur Ausgabe eines flüssigen Produkts, umfassend einen Behälter (10), der ein flüssiges Produkt enthält, einen Ausgabekopf (20), der mit einer Ausgabeöffnung (21) versehen ist, Ausgabemittel (25), um mindestens einen Teil des flüssigen Produkts durch die Ausgabeöffnung (21) hindurch auszugeben, und Durchlassmittel (40), um bei der Betätigung der Ausgabemittel (25) den Behälter (10) mit der Ausgabeöffnung (21) zu verbinden, wobei die Vorrichtung mindestens einen porösen Einsatz (50) umfasst, der aus porösem Material ausgeführt ist, um, insbesondere durch Kapillarwirkung, nach der Betätigung das restliche flüssige Produkt anzusaugen und mindestens teilweise aufzufangen, **dadurch gekennzeichnet, dass** der Behälter (10) einen Stopfen (25) umfasst, der den Behälter (10) vor der Betätigung dicht verschließt, wobei die Durchlassmittel (40) eine Nadel (41) umfassen, die geeignet ist, den Stopfen (25) bei der Betätigung zu durchstechen.

2. Vorrichtung nach Anspruch 1, bei der die Nadel (41) in Bezug auf die Ausgabeöffnung (21) feststeht.

3. Vorrichtung nach einem der vorhergehenden Ansprüche, bei welcher der poröse Einsatz (50) aus hydrophilem porösem Polymer ausgeführt ist.

4. Vorrichtung nach einem der vorhergehenden Ansprüche, bei welcher der poröse Einsatz (50) mindestens teilweise um die Durchlassmittel (40) herum angeordnet ist.

5. Vorrichtung nach Anspruch 4, bei welcher sich der poröse Einsatz (50) nach der Betätigung mindestens teilweise in dem Behälter (10) erstreckt.

6. Vorrichtung nach Anspruch 4 oder 5, bei der ein proximaler starrer Einsatz (60) stromab des porösen Einsatzes (50) angeordnet ist.

7. Vorrichtung nach Anspruch 6, bei welcher der proximale starre Einsatz (60) ein Zerstäubungsprofil direkt stromauf der Ausgabeöffnung (21) definiert.

8. Vorrichtung nach einem der Ansprüche 4 bis 7, bei der ein distaler starrer Einsatz (70) stromauf des porösen Einsatzes (50) angeordnet ist.

9. Vorrichtung nach Anspruch 8 und einem der Ansprüche 6 oder 7, bei welcher der poröse Einsatz (50) axial zwischen dem proximalen starren Einsatz (60) und dem distalen starren Einsatz (70) angeordnet ist.

10. Vorrichtung nach einem der Ansprüche 1 bis 3, bei welcher der poröse Einsatz (50) in dem Behälter (10) angeordnet ist.

11. Vorrichtung nach Anspruch 10, bei welcher der Behälter (10) einen zweiten Stopfen (26) enthält, der den porösen Einsatz (50) von dem in dem Behälter (10) enthaltenen flüssigen Produkt trennt.

12. Vorrichtung nach Anspruch 11 und einem der Ansprüche 2 oder 3, bei der die Nadel (41) am Ende der Betätigung den zweiten Stopfen (26) durchsticht, um den porösen Einsatz (50) mit den Durchlassmitteln (40) zu verbinden.

## Claims

1. A fluid dispenser device comprising: a reservoir (10) containing fluid; a dispenser head (20) that is provided with a dispenser orifice (21); dispenser means (25) for dispensing at least a fraction of said fluid through said dispenser orifice (21); and passage means (40) for connecting said reservoir (10) to said dispenser orifice (21) on actuation of said dispenser means (25), said device further comprising at least one porous insert (50) that is made out of porous material so as to suck up, in particular by capillarity, and to trap, at least in part, any residual fluid after actuation; the dispenser device being **characterized in that** said reservoir (10) includes a stopper (25) that closes said reservoir (10) in leaktight manner before actuation, said passage means (40) including a needle (41) that is adapted to pierce said stopper (25) during actuation.

2. A device according to claim 1, wherein said needle (41) is stationary relative to said dispenser orifice (21).

3. A device according to any preceding claim, wherein said porous insert (50) is made out of porous hydrophilic polymer.

4. A device according to any preceding claim, wherein said porous insert (50) is arranged, at least in part, around said passage means (40).

5. A device according to claim 4, wherein, after actuation, said porous insert (50) extends, at least in part, into said reservoir (10).

6. A device according to claim 4 or claim 5, wherein a proximal rigid insert (60) is arranged downstream from said porous insert (50).

7. A device according to claim 6, wherein said proximal rigid insert (60) defines a spray profile directly upstream from said dispenser orifice (21).

8. A device according to any one of claims 4 to 7, wherein a distal rigid insert (70) is arranged upstream from said porous insert (50).

9. A device according to claim 8 and according to claim 6 or claim 7, wherein said porous insert (50) is arranged axially between said proximal rigid insert (60) and said distal rigid insert (70).

10. A device according to any one of claims 1 to 3, wherein said porous insert (50) is arranged in said reservoir (10).

11. A device according to claim 10, wherein said reservoir (10) contains a second stopper (26) that separates said porous insert (50) from the fluid contained in said reservoir (10).

12. A device according to claim 11 and according to claim 2 or claim 3, wherein, at the end of actuation, said needle (41) pierces said second stopper (26) so as to connect said porous insert (50) to said passage means (40).
